(19) Europäisches Patentamt
European Patent Office
Office européen des brevets

(11) Publication number: **0 412 744 A1**

(12) **EUROPEAN PATENT APPLICATION**

(21) Application number: **90308611.4**

(51) Int. Cl.$^5$: **A61K 7/06**

(22) Date of filing: **06.08.90**

(30) Priority: **08.08.89 GB 8918080**

(43) Date of publication of application:
**13.02.91 Bulletin 91/07**

(84) Designated Contracting States:
**AT BE CH DE DK ES FR GB GR IT LI NL SE**

(71) Applicant: **UNILEVER PLC**
**Unilever House Blackfriars**
**London EC4P 4BQ(GB)**
(84) **GB**

Applicant: **Unilever N.V.**
**Burgemeester s'Jacobplein 1**
**NL-3015 CA Rotterdam(NL)**
(84) **BE**

(72) Inventor: **Gallagher, Peter**
**1 Mission Cottage, (off Moss Lane)**
**Burscough, Lancashire(GB)**
Inventor: **McGee, Thomas**
**74 Stanley Road**
**Hoylake, Wirral, Merseyside L47 LH2(GB)**
Inventor: **Khoshdel, Ezat**
**The Nook, Church Lane**
**Neston, Wirral, Merseyside L64 9US(GB)**

(74) Representative: **Ford, Michael Frederick et al**
**MEWBURN ELLIS 2 Cursitor Street**
**London EC4A 1BQ(GB)**

(54) **Hair treatment composition.**

(57) A hair treatment composition contains a derivative of a polymer complex of polyaminoglucose and glycan. The derivative is obtainable through reaction with an electrophile to introduce aliphatic or aromatic substitution on oxygen or nitrogen so that the derivative is soluble in a hydrogen bonded solvent.

EP 0 412 744 A1

# HAIR TREATMENT COMPOSITION

## FIELD OF THE INVENTION

The present invention relates to a hair treatment composition, and more particularly to a hair treatment composition containing a derivative of polyaminoglucose glycan polymer complex (PAGGPC) which is soluble in an aqueous based surfactant system.

## BACKGROUND OF THE INVENTION

Water soluble salts of PAGGPC may be used as the film-former in hair setting compositions. Our earlier patent application, case J3115, filed in EPO as 90306499.6 discloses the use of such salts in aqueous and aqueous ethanolic hair setting compositions. However, in order for the material to be effective as a film-former, it must remain protonated, and so the pH of the composition must be low.

PAGGPC may be obtained by treating the mycelia of certain fungal species, such as Aspergillus niger, with strong alkali, as described in for example GB-A-2026516 (Muzzarelli) or US-A-4806474 (Miles). Aspergillus niger is a common waste product in industrial fermentation processes, for example in the production of citric acid.

Polyaminoglucose glycan polymer complex has not been fully characterized, but NMR studies have shown that it is distinct in character from chitosan, and physical comparisons show that films formed by such a complex and by chitosan are different from each other. Polyaminoglucose glycan polymer complex is commercially available, for example as RIOSAN (Trademark) from Meyhall.

The introduction of PAGGPC into other hair products such as shampoos and conditioners enables the user to obtain lasting hold and set and increased volume to the hair.

However use of the water-soluble salts referred to above in shampoo or conditioner systems is undesirable because of the need to keep the pH below 7, preferably below 4. Such low pH values in these systems introduce problems of formulation, as many of the conventional surfactants become less stable at low pH, as well as the possibility of skin irritation due to the low pH, which may also be experienced by some users.

We have found that special derivatives of PAGGPC which are soluble in hydrogen-bonded solvents may be incorporated into hair treatment compositions. Since these derivatives are soluble in shampoos and conditioners without the need to drastically reduce the pH, the problems of stability and skin irritation will be avoided.

## BRIEF SUMMARY OF THE INVENTION

Accordingly, the invention provides a hair treatment composition comprising a derivative of polyaminoglucose glycan polymer complex which is dissolved in a hydrogen-bonded solvent. The composition is preferably in the form of a shampoo, a conditioner or a mousse.

## DETAILED DESCRIPTION OF THE INVENTION

### Derivatives of PAGGPC

The derivatives of PAGGPC which are used in the hair treatment compositions of the invention enable the hair to be set and to retain its shape and give improved volume. These PAGGPC derivatives also confer some conditioning and shine benefit on the hair.

The derivatives are preferably the product of the reaction of PAGGPC with an electrophile.

The electrophile is suitably chosen from epoxides, $C_{1-30}$ alkyl halide, $C_{1-30}$ acyl halide, polyethylene glycol halide, acid anhydride, sultones, alkyl lactones, $C_{1-30}$ alkyl ester, aryl ester or $C_{1-30}$ aliphatic aldehyde.

Preferred examples of epoxides which may be used as the electrophile include -

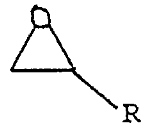

I

where R is chosen from H, $C_{1-20}$ alkyl, $C_{3-6}$ cycloalkyl, aryl, $-CH_2OR^1$, and $R^1$ is H, $C_{1-20}$ alkyl, $C_{3-6}$ cycloalkyl or aryl, or

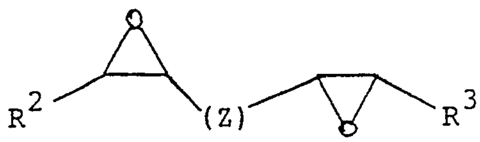

II

where $R^2$ and $R^3$ are the same or different and are chosen from H or $C_{1-20}$ alkyl, and Z is aryl, $C_{1-30}$ saturated or unsaturated alkyl or $C_{2-30}$ ethoxylated alkyl, or derivatives thereof.

Examples of suitable halides include 1-bromoethane, 1-bromohexane, 1-chlorododecane, chloropolyethylene glycol, ethanoyl chloride, propionyl choride, benzoyl chloride and stearoyl chloride. Suitable acid anhydrides include acetic, succinic, maleic, phthalic and citric anhydrides, or derivatives thereof.

Sultones which may be used as the electrophile are for example 1,3-propane sultone and 1,4-butane sultone, and suitable esters include ethyl ethanoate, ethyl benzoate and benzoyl benzoate.

The composition of the invention may comprise from 0.01 to 25% by weight of the derivative of PAGGPC, preferably from 0.1 to 5% by weight.

PAGGPC will normally contain free hydroxyl groups and some acetylated amine groups. Under suitable conditions the electrophile, from those outlined above, will add to the hydroxyl group or groups. However, reaction is often easier, and preferable, if the PAGGPC is deacetylated by treating with strong alkali. The electrophile, under suitable conditions, will then react with both the free amine and the hydroxyl groups, although the electrophile will preferentially attack the amine group(s).

It should be noted that for certain electrophiles eg 1,2-epoxypropane, moderately to high levels of substitution are required to obtain a derivative of PAGGPC which is soluble in the hydrogen-bonded solvents used in the compositions of the invention. Low levels of substitution give rise to derivatives which are insoluble in the hydrogen-bonded solvents. Such derivatives would not be suitable for use in the compositions of the invention. The person skilled in the art will readily ascertain the required degree of substitution for any given electrophile.

The use of derivatives having low levels of substitution is the subject of our co-pending application J.3118 having the same date as this application.

## Hydrogen-Bonded Solvent

The hair treatment composition according to the invention further comprises a hydrogen-bonded solvent, preferably in an amount of from 1 to 50% by weight of the composition.

Examples of suitable hydrogen bonded solvents include water, aqueous surfactants or aqueous alcohol. Further examples of hydrogen-bonded solvents can be found in part C of Table II at page C-696 to C-698 of the Handbook of Chemistry and Physics, 1983-4, 64th Edition, Ed. Robert C Weast, CRC Press.

## Form of the composition

The hair treatment composition of the invention may be in the form of a shampoo. It will then preferably further comprise a surfactant, which is chosen from anionic, nonionic and amphoteric surfactants, and mixtures thereof.

Suitable anionic surfactants are the alkyl sulphates, alkyl ether sulphates, alkaryl sulphonates, alkyl succinates, alkyl sulphosuccinates, N-alkoyl sarcosinates, and alpha-olefin sulphonates, especially their sodium, magnesium, ammonium and mono-, di- and triethanolamine salts. The alkyl groups generally contain from 8 to 18 carbon atoms and may be unsaturated. The alkyl ether suphates may contain from 1 to 10 ethylene oxide or propylene oxide units per molecule, and preferably contain 2 to 3 ethylene oxide units

per molecule.

Examples of suitable anionic surfactants include sodium lauryl sulphate, sodium oleyl succinate, ammonium lauryl sulphosuccinate ammonium lauryl sulphate, sodium dodecylbenzene sulphonate, triethanolamine dodecylbenzene sulphonate and sodium N-lauryl sarcosinate. The most preferred anionic surfactants are sodium lauryl sulphate, sodium lauryl ether sulphate 2EO and 3EO, ammonium lauryl sulphate and ammonium lauryl ether sulphate 1EO, 2EO and 3EO.

The nonionic surfactants suitable for use in the composition of the invention may include condensation products of aliphatic ($C_{8-18}$) primary or secondary linear or branched chain alcohols or phenols with alkylene oxides, usually ethylene oxide and generally 6-30 EO.

Other suitable nonionics include mono or di alkyl alkanolamides or alkyl polyglucosides. Examples include coco mono or diethanolamide, coco mono isopropanolamide, and coco di glucoside.

The amphoteric surfactants suitable for use in the composition of the invention may include alkyl amine oxides, alkyl betaines, alkyl amidopropyl betaines, alkyl sulphobetaines, alkyl glycinates and alkyl carboxyglycinates, wherein the alkyl groups have from 8 to 18 carbon atoms. Examples include lauryl amine oxide, cocamidopropyl betaine, cocodimethyl sulphopropyl betaine and preferably cocobetaine.

The composition of the invention when it is in the form of a shampoo preferably comprises from 2 to 40% by weight, and preferably from 5 to 30% by weight of the surfactant.

If an amount of less than 2% by weight of surfactant is present, inadequate foaming is achieved, and if more than 40% by weight is present, no further increase in cleansing power or foaming ability is observed.

The hair treatment composition of the invention may alternatively be in the form of a hair conditioning composition, and will then preferably comprise a conditioning agent.

Suitable conditioning agents include cationic surfactants, cationic polymers, quaternised silicones, volatile silicones, protein hydrolysates or quaternised protein hydrolysates.

Examples of cationic surfactants include -

Cetyl trimethylammonium chloride
Stearyl dimethylbenzyl ammonium chloride
Cetylpyridinium chloride
Quaternium -5
Quaternium -31
Quaternium -18
and mixtures thereof.

Suitable cationic polymers include -

Guar Hydroxypropyltrimonium chloride
Quaternium -19
Quaternium -23
Quaternium -40
Quaternium -57
Poly(dimethyldiallylammonium chloride)
Poly(dimethylbutenyl ammonium chloride)-$\alpha$ $\omega$ -bis(triethanolammonium chloride)
Poly(dipropyldiallylammonium chloride)
Poly(methyl-$\beta$-propaniodiallylammonium chloride)
poly(diallylpiperidinium chloride)
Poly(vinyl pyridinium chloride)
Quaternised poly (vinyl alcohol)
Quaternised poly (dimethylaminoethylmethacrylate) and mixtures thereof.

Examples of suitable volatile silicone materials include cyclomethicone available commercially as Dow Corning DC 345 and Volatile Silicone 7158, available from Union Carbide.

The quaternised silicones which may be used in the compositions of the invention are generally amino functional polydimethylsiloxanes. Examples include amodimethicone available commercially as Dow Corning DC 929 and trimethylsilylamodimethicone, available as Dow Corning Q 8220.

Suitable protein hydrolysates include lauryl dimonium hydroxy propylamino hydrolysed animal protein, available commercially under the trade name LAMEQUAT L, and hydrolysed keratin containing sulphur-bearing amino acids, available commercially under the trade name CROQUAT WKP.

When the composition of the invention is in the form of a hair conditioning composition, it preferably comprises from 0.01 to 10% by weight of the conditioning agent.

The hair treatment composition of the invention may also be a mousse, which is a leave-on product used after shampooing the hair. The mousse delivers the PAGGPC complex onto the hair where it holds the hair set.

Mousse compositions according to the invention will preferably further comprise a surfactant such as lauryl dimethylamine oxide or lauryl betaine, and a propellant gas such as hydrocarbon, fluorocarbon, chlorofluorocarbon or compressed gas, for example air, nitrogen or carbon dioxide.

The derivatives of PAGGPC used in the compositions according to the invention are insoluble in the liquified propellant hydrocarbon, fluorocarbon or chlorofluorocarbon gases used in the mousse compositions, and sufficient quantities of hydrogen-bonded solvent must be present in the composition to dissolve the derivatives.

Other ingredients

The hair treatment composition of the invention nay also comprise other ingredients known in the art including viscosity control agents, solubility control agents, foam boosters, opacifiers, perfumes, colouring agents, antidandruff agents, preservatives, proteins, polymers, buffering agents, thickening agents.

The invention is further illustrated by the following Examples.

EXAMPLES

Preparation of PAGGPC derivatives

Preparation Example A

PAGGPC was N-propoxylated by heating deacetylated PAGGPC in a sealed tube (or an autoclave) with propylene oxide in an aqueous ethanol solvent for 12 hours at 100 °C, to give N-hydroxypropyl PAGGPC (A).

Preparation Example B

The N-hydroxypropyl PAGGPC derivative obtained in Preparation Example A above was further functionalised by heating at between 60 and 80 °C in an autoclave with 1-bromopropane in acetone in the presence of NaOH for 18 hours. O-propyl,N-hydroxypropyl PAGGPC (B) was obtained.

Preparation Example C

Crude, acetylated PAGGPC was heated in dimethyl formamide (DMF) at 100 °C for 15 hours in the presence of 1-bromooctane to give O-octyl PAGGPC (C).

Preparation Example D

Deacetylated PAGGPC was heated in DMF with 1-bromohexane at 80 °C for 15 hours to give N-hexyl PAGGPC (D).

Example 1

The following is an example of a shampoo according to the invention:

| | % w/w |
|---|---|
| SLES 2EO | 15.0 |
| Cocodiethanolamide | 1.0 |
| PAGGPC (A) | 1.0 |
| NaCl | 0.5 |
| Preservative/perfume/colour | qs |
| Water to | 100 |

Example 2

The following is an example of a shampoo according to the invention:

| | % w/w |
|---|---|
| SLES 2EO | 12.0 |
| Cocodiethanolamide | 3.0 |
| Jaguar C-13S[1] | 0.3 |
| NaCl | 0.5 |
| PAGGPC (D) | 1.0 |
| Perfume/colour/preservative | qs |
| Water to | 100 |

1 - Jaguar C-13S is guar hydroxypropyltrimonium chloride.

Example 3

The following is an example of a hair conditioning composition according to the invention.

| | % w/w |
|---|---|
| Cetyl trimethylammonium chloride | 0.7 |
| Cetostearyl alcohol | 2.0 |
| Paraffin wax | 1.0 |
| Glycerol monostearate | 0.7 |
| PAGGPC (B) | 1.5 |
| Preservative/perfume/colour | qs |
| Water to | 100 |

Example 4

The following is an example of a hair conditioning composition according to the invention:

| | % w/w |
|---|---|
| Cetyl trimethylammonium chloride | 0.7 |
| Ceyostearyl alcohol | 1.0 |
| Natrosol 250 HR [2] | 1.3 |
| PAGGPC (C) | 1.3 |
| Water to | 100 |

2 - Natrosol 250 HR os hydroxyethyl cellulose

Example 5

The following is an example of a mousse composition according to the invention:

| | % w/w |
|---|---|
| PAGGPC (C) | 1.5 |
| Ethanol | 5.0 |
| Hydrocarbon propellant | 7.5 |
| Empigen OB [3] | 0.03 |
| Silicone glycol | 0.04 |
| Perfume | q.s. |
| Arquad 16/50 [4] | 0.08 |
| Water to | 100 |

3 - Empigen OB is lauryl dimethylamine oxide
4 - Arquad 16.50 is a mixture of cetyl trimethylammonium chloride and isopropanol.

## Claims

1. A hair treatment composition comprising a derivative of polyaminoglucose glycan polymer complex dissolved in a hydrogen-bonded solvent.
2. A hair treatment composition as claimed in Claim 1 wherein the derivative of polyaminoglucose glycan polymer complex comprises the reaction product of the polymer complex with an electrophile.
3. A hair treatment composition as claimed in Claim 2 wherein the polyaminoglucose glycan polymer complex is bonded through nitrogen or oxygen to residues of the electrophile which are aliphatic or aromatic groups containing one to thirty two carbon atoms,
4. A hair treatment composition as claimed in any one of Claims 1,2 or 3 wherein the electrophile is chosen from $C_{2-23}$ expoxides, $C_{5-74}$ bis epoxides, $C_{1-30}$ alkyl halide, $C_{1-30}$ acyl halide, polyethylene glycol halide, acid anhydride, sultones, alkyl lactones, $C_{1-30}$ alkyl ester, aryl ester and $C_{1-30}$ aliphatic aldehyde.
5. A hair treatment composition as claimed in claim 4 wherein the expoxide is chosen from

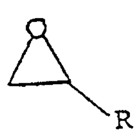

I

where R is chosen from H, $C_{1-20}$ alkyl, $C_{3-6}$ cycloalkyl, aryl, $-CH_2OR^1$, and $R^1$ is H, $C_{1-20}$ alkyl, $C_{3-6}$ cycloalkyl or $C_{1-6}$ aryl, or

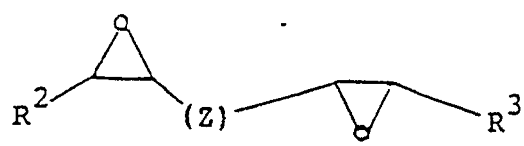

II

where $R^2$ and $R^3$ are the same or different and are chosen from H or $C_{1-20}$ alkyl, and Z is aryl, $C_{1-30}$ saturated or unsaturated alkyl or $C_{2-30}$ ethoxylated alkyl.

6. A hair treatment composition as claimed in claim 4 wherein the acid anhydride is chosen from acetic, succinic, maleic, phthalic, citric anhydrides or derivatives thereof.

7. A hair treatment composition as claimed in any preceding claim wherein the derivative of PAGGPC is present in an amount of from 0.01 to 25% by weight.

8. A hair treatment composition as claimed in any preceding claim which is in the form of a shampoo.

9. A hair treatment composition as claimed in any one of claims 1 to 7 which is in the form of a hair conditioning composition.

10. A hair treatment composition as claimed in any one of claims 1 to 7 which is in the form of a mousse.

European Patent Office

# EUROPEAN SEARCH REPORT

Application Number

EP 90308611.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US - A - 4 780 310 (G. LANG et al.) * Claims 1,2; column 2, line 60 - column 4, line 26 * | 1-10 | A 61 K 7/06 |
| X | US - A - 4 765 976 (J.F. GROLLIER et al.) * Totality * | 1-10 | |
| X | EP - A1 - 0 297 455 (CRINOS INDUSTRIA) * Claims 1-3; abstract * | 1 | |
| X | DE - A1 - 3 632 030 (WELLA AG) * Abstract; page 4, lines 30,31 * | 1,7 | |
| A | FR - A - 2 094 109 (I.I. LUBOWE) * Page 2, lines 21-35; page 4, lines 16-26 * | 1 | TECHNICAL FIELDS SEARCHED (Int. Cl.5) |
| A | US - A - 3 632 754 (L.L. BALASSA) * Totality * | 1 | A 61 K 7/00 |

The present search report has been drawn up for all claims

| Place of search | Date of completion of the search | Examiner |
|---|---|---|
| VIENNA | 23-11-1990 | IRMLER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)